# EUROPEAN PATENT APPLICATION

(11) **EP 4 755 487 A1**
(43) Date of publication of application: **10.06.2026**
(21) Application number: 24217494.4
(22) Date of filing: 04.12.2024
(51) Int. Cl.: B01D 15/20, B01D 15/42, B01D 61/00, C07K 1/16, C12N 15/00, G06F 17/00, B01J 20/30, G01N 30/00

(54) **PROCESSING METHODS FOR BIOLOGICAL PRODUCTS INVOLVING PULSES**

(71) Applicant: Sartorius BIA Separations d.o.o., 5270 Ajdovscina (SI)
(72) Inventor: Cernigoj, Urh, 5270 Ajdovscina (SI); Strancar, Ales, 5270 Ajdovscina (SI); Sever, Tadej, 5270 Ajdovscina (SI)
(74) Representative: Cohausz & Florack

(57) **Abstract**

The invention relates to a method for processing a biological product wherein the method involves pulses. The invention also concerns a system configured to perform this method. Further, the invention concerns the use of pulsing in a method for processing a biological product and the use of a bypass to enable pulsing in a method for processing a biological product. Lastly, the invention relates to a computer program product causing a computer to at least transmit a command to a pulsing unit employed in a system configured to perform a method for processing a biological product.

## Description

### FIELD OF THE INVENTION

The invention relates to a method for processing a biological product involving pulses. The invention also concerns a system configured to perform this method. Further, the invention concerns the use of pulsing in a method for processing a biological product and the use of a bypass to enable pulsing in a method for processing a biological product. Lastly, the invention relates to a computer program product causing a computer to at least transmit a command to a pulsing unit employed in a system configured to perform a method for processing a biological product.

### BACKGROUND OF THE INVENTION

Biological products such as viruses, virus-like particles, vesicles, exosomes, antibodies or nucleic acids become more and more important as biopharmaceuticals. During upstream production, biological products are usually produced by cell cultures of bacteria, yeast, plant or animal cells in bioreactors. To ensure high quality and safety of the manufactured biological products, downstream processing is performed. This typically includes the separation of the biological product from undesirable host cell fragments and host cell components such as DNA, RNA, proteins and lipids.

The methods forming part of downstream processing often involve porous media, e.g., in form of a membrane, a resin, or a monolithic material. These materials are usually used as solid phase supports, with which the biological products come into contact and interact. Different interaction strength between the solid phase support and the different biological molecules result in an ability to separate these molecules. A common characteristic of these porous media is the interstitial pore space, e.g., convective channels, where the process stream flows through due to the use of an external force, e.g., a pump. When large bioparticles such as viruses, virus-like particles, RNA and DNA are purified on such porous media, it often happens that the diameter of the constrictions in the interstitial pore space is in the range of biomolecule size. A certain amount of biological product and/or impurities is/are therefore convectively entrapped even under non-binding conditions. For the potential reuse of these porous media and thus their overall lifetime it is crucial that the porous media are cleaned efficiently from biological product and/or impurities. To achieve an optimal recovery rate of biological product, it is also important to completely elute the biological product from these porous media.

To avoid or at least reduce convective entrapment in porous media during downstream processing, a main strategy was so far to reduce the applied flow rate (Trilisky, Egor I., and Abraham M. Lenhoff. "Flow-dependent entrapment of large bioparticles in porous process media." *Biotechnology and bioengineering* 104.1 (2009): 127-133)). This approach, however, does not hinder convective entrapment completely and is also impractical due to the long processing times.

In summary, there is a need in downstream processing for obtaining a high yield of purified biological product, while at the same time a long lifetime of the employed equipment is made possible. There is in particular a need in downstream processing involving the use of a porous medium to obtain high yields of purified biological product and to enable a long reuse of the porous medium. Specifically, there is a need in downstream processing applying a porous medium to efficiently remove entrapped biological product and impurities while at the same time keeping the overall downstream process practical, in particular time-efficient.

### SUMMARY OF THE INVENTION

Against the above-described background, it was an objective of the present invention to provide an improved method for purifying a biological product, in particular to provide an improved method for purifying a biological product at high yield and purity. It was also an objective of the present invention to provide a method for purifying a biological product allowing the production of a biological product at high yield and purity, while at the same time a long lifetime of the employed equipment is ensured and the method remains practical, especially time-efficient.

The above-described problems are solved by the method according to claim 1, the system according to claim 9, the uses according to claims 10 and 13 as well as the computer program product according to claim 14.

The method according to the invention for processing a biological product comprises at least the following steps:
a) Providing a processing material comprising the biological product;
b) Purifying the processing material of step a) involving
   b.1) a flow of the processing material of step a) through a porous medium, and
   b.2) a flow of a process liquid through the treated porous medium of step b.1), wherein pulsing is performed,
wherein the pulsing of step b.2) is carried out according to one or more of the following options:
i. changing at least two times, preferably periodically, the flow rate of the process liquid through the treated porous medium , wherein the altered flow rate is achieved by allowing the process liquid to flow through a bypass around the treated porous medium;
ii. applying at least two times, preferably periodically, an external force; and
iii. switching at least two times, preferably periodically, the process liquid; and
iv. switching at least two times, preferably periodically, two or more parameters of the purification.

The research underlying the invention surprisingly demonstrated that pulsing during the flow of a process liquid through a porous medium already treated with processing material comprising a biological product has advantageous effects: Firstly, the overall recovery rate of biological product can be increased. Without being bound by theory, this seems to be due to the effect that the pulsing helps to release convectively entrapped biological product from pore constrictions of the porous medium. Even under non-binding conditions, a certain portion of biological product typically gets entrapped in pore constrictions of the porous medium. The pulsing supports the diffusive escape of entrapped biological product from these pore constrictions and therefore helps to increase the overall recovery rate of biological product. This works particularly well, when at least pulses of option i are used. Secondly, the separation efficiency between the biological product and impurities is improved. Not only biological product, but also impurities get convectively entrapped in porous media. The pulsing can be used to remove as many impurities as possible from the employed porous medium before the elution of biological product from the porous medium is triggered. This also works particularly well, when at least pulses of option i are used. Thirdly, biological products can exist in different isoforms with varying spatial dimensions (e.g., plasmid DNA can exists in the isoforms supercoiled, open circular and linear). One of these isoforms is typcially particularly susceptible to convective entrapment. The release of convectively entrapped biological product thus often allows at the same time to isolate biological product of the same isoform. This works particularly well, when at least pulses of option i are used. Fourthly, the cleaning and as a consequence also the lifetime of the porous medium can be improved by the pulsing. Without being bound by theory, the pulsing allows to remove biological product and impurities from the porous medium very efficiently. In that way, the porous medium is optimally regenerated for subsequent use. Again, this works particularly well, when at least pulses of option i are used.

The invention further comprises a system configured to perform the method of the invention.

The system allows to perform the method of the invention with all its advantages.

The invention also comprises the use of pulsing in a method for processing a biological product, wherein the method for processing the biological product comprises at least the steps of
a) Providing a processing material comprising the biological product;
b) Purifying the processing material of step a) involving
   b.1) a flow of the processing material of step a) through a porous medium, and
   b.2) a flow of a process liquid through the treated porous medium of step b.1), wherein pulsing is performed,
   wherein the pulsing of step b.2) is performed according to any one of the following options
   i. changing at least two times, preferably periodically, the flow rate of the process liquid through the treated porous medium, wherein the altered flow rate is achieved by allowing the process liquid to flow through a bypass around the treated porous medium;
   ii. changing at least two times, preferably periodically, two or more parameters of the method for processing a biological product;
   iii. applying at least two times, preferably periodically, an external force; and
   iv. changing at least two times, preferably periodically, the process liquid;
      and wherein the pulsing is used for
      - improving the cleaning of the porous medium;
      - improving the lifetime of the porous medium;
      - improving the purity of the biological product by improving the separation efficiency between the biological product and impurities; and/or
      - improving the recovery of the biological product from the porous medium.

Without being bound by theory, the pulses applied in the method for processing a biological product help to release convectively entrapped biological product and impurities by diffusive escape. In that way, the cleaning of the porous medium, the lifetime of the porous medium, the purity of the biological product and the recovery of the biological product from the porous medium can be improved.

The invention also concers the use of a bypass to enable a pulsing of an applied flow rate during a purification method for purifying a biological product involving a process flow through a porous medium for separation, wherein the bypass allows to guide the process flow around the porous medium. The inventors found that the use of a bypass in this context has several advantages: By using a bypass to enable pulsing of an applied flow rate, it is possible to maintain a constant flow of process liquid in the subsequent flow path downstream of the bypass/the bypassed flow path. This is particularly advantegous for subsequent detector measurements. Moreover, a bypass makes it possible that the pump can run constantly without the need of a "ramp-up" phase to establish the desired flow rate. This ensures a constant flow rate and thus makes the pulses more precise.

Lastly, the invention concerns a computer program product comprising instructions, which, when the program is executed by a computer, cause the computer to carry out at least the step of transmitting a command to a pulsing unit employed in a system configured to perform a method for processing a biological product comprising the steps of
a) Providing a processing material comprising the biological product; and
b) Purifying the processing material of step a) comprising
   b.1) a flow of the processing material of step a) through a porous medium, and
   b.2) a flow of a process liquid through the treated porous medium of step b.1), wherein pulsing is performed,
wherein the command causes the pulsing unit to perform a pulsing according to one or more of the following options:
i. switching at least two times, preferably periodically, the flow rate of the process liquid through the treated porous medium, wherein the altered flow rate is achieved by allowing the process liquid to flow through a bypass around the treated porous medium;
ii. switching at least two times, preferably periodically, two or more parameters of the purification;
iii. applying at least two times, preferably periodically, an external force; and
iv. switching at least two times, preferably periodically, the process liquid.

The computer program product according to the invention enables pulsing in a system configured to perform the method according to the invention.

### DETAILED DESCRIPTION OF ILLUSTRATIVE EMBODIMENTS

### The method

The method of the invention for processing a biological product comprises at least the following steps:
a) Providing a processing material comprising the biological product;
b) Purifying the processing material of step a) involving
   b.1) a flow of the processing material of step a) through a porous medium, and
   b.2) a flow of a process liquid through the treated porous medium of step b.1), wherein pulsing is performed,
wherein the pulsing of step b.2) is carried out according to one or more of the following options:
i. changing at least two times, preferably periodically, the flow rate of the process liquid through the treated porous medium, wherein the altered flow rate is achieved by allowing the process liquid to flow through a bypass around the treated porous medium;
ii. applying at least two times, preferably periodically, an external force;
iii. switching at least two times, preferably periodically, the process liquid; and
iv. switching at least two times, preferably periodically, two or more parameters of the purification.

Additional steps can be included in the method of the invention. In particular, additional steps can be included before step a), in between steps a) and b), in between steps b.1) and b.2) and after step b).

In step a) of the method of the invention, a processing material comprising a biological product is provided. The processing material which is treated in the method of the invention comprises the biological product. The processing material can also comprise impurities. Such impurities can be, for example, medium components and/or host cell components such as proteins, DNA, RNA and lipids. The term "processing material" as used herein means a composition comprising the biological product and is suitable for purification involving a porous medium. The processing material can be the feed material provided from upstream processing or can be an intermediate material generated during previous downstream processing. The terms "upstream processing" and "downstream processing" are known to the skilled person. Upstream processing typically relates to all the processes related to the production of a biological product, e.g., by cell cultures of bacteria, yeast, plant and or animal cells in bioreactors. Downstream processing typically refers to all the processes serving to purify, enrich and/or prepare the biologicial product for further use, in particular further medical use.

The term "biological product" means any biological particle that is obtained as desired product from a biomanufacturing process. In one embodiment, the biological product is selected from the group consisting of a nucleic acid, a bacterium, a eukaryotic cell, a virus, a virus-like particle, a cell-derived vesicle, a protein, and mixtures or complexes thereof.

The term "nucleic acid" as used herein encompasses any kind of RNA and DNA. A nucleic acid in form of DNA includes in particular plasmid DNA and its isoforms (supercoiled, open circular or linearized).

The term "mixture of biological particles" relates to a composition containing at least two different biological particles selected from the group consisting of a nucleic acid, a virus, a bacterium, a eukaryotic cell, a virus-like particle, a cell-derived vesicle, and a protein. In a mixture, the contained different biological particles typically do not form stable associations. In contrast, the term "complex of biological particles" relates to a composition comprising stable associations of biological particles wherein the complexes can contain the same or different biological particles selected from the group consisting of a nucleic acid, a virus, a virus-like particle, a cell-derived vesicle, and a protein.

In one embodiment, the biological product is a nucleic acid. Experiments of the inventors have shown that pulsing is particularly effective in methods for processing nucleic acids as biological product, especially plasmid DNA. Plasmid DNA can exists in different isoforms (supercoiled, open circular or linearied) and can thus have different spatial conformations. Due to their different spatial conformations, the different plasmid DNA isoforms are also effected differently by convective entrapment upon use of a particular porous medium. The open circular plasmid DNA isoform is particularly prone to convective entrapment. The pulsing applied in the method of the invention helps to release entrapped plasmid DNA molecules from the pore constrictions of the porous medium and therefore increases the overall recovery rate of plasmid DNA. This works particularly well, when at least pulses of option i are used. Advantageous of the fraction released from convective entrapment with the aid of pulsing is also that the released molecules typically have the same isoform. With regard to plasmid DNA used as biological product this means that pure open circular plasmid DNA can be isolated by the method of the invention.

The term "virus" as used herein relates to any type of natural or artificially created virus.

The term "virus-like particle" relates to delivery vesicles that mimic viruses, but are non-infectious because of the absence of viral genetic material.

The term "cell-derived vesicle" as used herein refers to vesicles originating from cells. The vesicles can originate from any type of cells.

The term "protein" as used herein relates to any natural or artificial biomolecule comprising one or more long chains of amino acid residues.

The term "bacterium" is known to the skilled person and relates to any kind of natural or engineered bacterium.

The term "eukaryotic cell" is known to the skilled person and relates to any kind of natural or engineered eukaryotic cell.

In step b) of the method of the invention, the processing material of step a) is purified. The term "the processing material of step a) is purified" means that "the processing material of step a) is treated so that the biological product contained in the processing material is enriched and/or separated from other components of the processing material". Other components of the processing material can be in particular unwanted impurities.

The purification method of step b) can be any purification method suitable to purify a biological product wherein the purification method involves a porous medium as separation medium. The skilled person knows based on his or her common general knowledge suitable purification methods of this kind. The purification method can, for example, be a chromatography separation method, a tangential flow filtration method, an extraction method, a precipitation method or a filtration method.

In one embodiment, the porous medium is selected from the group consisting of a membrane, a resin, fibers and a monolithic material. The skilled person knows based on his or her common general knowledge which porous medium is suitable for which kind of purification method to purify a certain biological product. The membrane can be any kind of porous membrane suitable to purify a biological product. Preferably the membrane has a thickness of 50 to 300 µm. The resin can be any type of medium suitable to purify a biological product. The resin typically contains a matrix serving as the backbone of the resin, wherein the resin is optionally functionalized with appropriate chemical groups to enable a certain purification mode. The resin matrix can be made from various materials like polymers, silica, or agarose. The monolithic material can be any kind of one-piece medium suitable to purify a biological product. In other words, the monolithic material can be any kind of single-unit medium suitable to purify a biological product. The fibers can be any type of fibers suitable to purify a biological product. The fibers can be of natural or synthetic origin. The fibers can be used in different formats, e.g., as staple, whole fabrics, and aligned fibers.

In one embodiment, the purification method is a chromatography separation method, wherein the porous medium is the stationary phase. Experiments of the inventors have shown that pulsing applied in a chromatography separation method allows to remove convectively entrapped molecules from the stationary phase very efficiently, especially when at least pulses of option i are used. This ultimately leads to the following advantages: Firstly, the overall recovery rate of biological product from the stationary phase can be increased. Secondly, the separation efficiency between the biological product and impurities can be improved. Not only biological product, but also impurities contained in the processing material get convectively entrapped in the stationary phase. The pulsing can be used to remove as many impurities as possible from the employed stationary phase before the elution of biological product from the stationary phase is triggered. Thirdly, the to be separated biological products can exists in different isoforms with varying spatial dimensions. The release of convectively entrapped biological product therefore often allows at the same time to isolate another fraction of biological product that has the same isoform. Fourthly, the cleaning and as a consequence also the lifetime of the stationary phase can be improved by the pulsing because the porous medium can be optimally regenerated for subsequent use.

The term "chromatography separation method" as used herein means any chromatography separation method which allows the separation of the processing material by different distribution of the processing material components between a stationary phase and a mobile phase. The chromatography separation method can, for example, be an ion exchange chromatography separation method, a size exclusion chromatography separation method, a reverse phase chromatography separation method, a hydrophobic interaction chromatography separation method, an affinity chromatography separation method or a mixed mode chromatography separation method. The skilled person knows based on his or her common general knowledge which kind of chromatography separation method is suitable for a particular biological product.

The stationary phase can be any phase suitable for a chromatography separation method. The stationary phase can, for example, be a resin, a membrane adsorber, fibers or a monolithic material.

The stationary phase in form of a resin typically contains a matrix serving as the backbone of the resin, wherein the resin is optionally functionalized with appropriate chemical groups or biological moieties to enable a certain purification mode. A biological moiety, for example, can be protein A. The resin matrix can be made from various materials like polymers, silica, or agarose and can also have the form of beads. A membrane adsorber as stationary phase is typically a membrane with pores. Preferably the membrane adsorber has a thickness of 50 to 300 µm. A monolithic material as stationary phase typically comprises a one-piece porous medium and thus a single-unit structure.

Step b) involves at least the steps b.1) and b.2).

Step b.1) encompasses a flow of the processing material of step a) through a porous medium. The term "flow of the processing material of step a) through a porous medium" refers to any flow of the processing material of step a) through a porous medium so that the biological product contacts the porous medium. In particular, the term "flow of the processing material of step a) through a porous medium" refers to any flow of the processing material of step a) through a porous medium, wherein the biological product is loaded onto the porous medium.

Step b.2) encompasses a flow of process liquid through the treated porous medium of step b.1), wherein pulsing is performed.

The term "treated porous medium of step b.1)" refers to the porous medium of step b.1) which has been contacted with biological product.

The term "flow of process liquid through the treated porous medium" means that the process liquid is contacted with the treated porous medium.

The process liquid is different from the processing material comprising the biological product. The process liquid can be any kind of process liquid suitable to support the purification of the biological product in the purification method of step b).

In one embodiment, the process liquid is substantially free or devoid of the biological product. "Substantially free of the biological product" means that the process liquid contains less than 1 % w/v of biological product, preferably less than 0.1 % w/v of biological product. Alternatively, "substantially free of biological product" means that the process stream contains less than 5 %, preferably less than 1 % of biological product as compared to the processing material comprising the product as used in step a).

In one embodiment, the process liquid is a washing solution. The term "washing solution" as used herein refers to any kind of washing solution serving to remove any unwanted material from the porous medium before the next step to purify the biological product from the porous medium is initiated.

In one embodiment, the process liquid is a cleaning solution. The term "cleaning solution" as used herein refers to any kind of cleaning solution serving to remove any unwanted material from the porous medium after the biological product has already been purified from the porous medium.

In one embodiment, the process liquid is an elution solution. The term "elution solution" as used herein refers to any kind of elution solution serving to release and/or mobilize the biological product from the porous medium.

In one embodiment, the purification method of step b) is chromatography separation method and the process liquid is selected from the group consisting of a washing solution, an elution solution and a cleaning solution. In one embodiment, the purification method of step b) is a chromatography separation method and the process liquid is a washing solution. The skilled person knows based on his or her common general knowledge which washing solution is appropriate for the applied kind of chromatography separation method and porous medium. The pulsing during the flow of a washing solution through the treated porous medium, i.e. the porous medium of step b.1) that has been contacted with the processing material containing the biological product, allows to efficiently remove unwanted material such as impurities from the porous medium. Without being bound by theory, the pulsing seems to support the diffusive escape of convectively entrapped unwanted material such as impurities. In that way, the separation efficiency between unwanted material and the biological product is further increased and even more pure biological product can be obtained with the method of the invention. In one embodiment, the purification method of step b) is a chromatography separation method and the process liquid is an elution solution. The pulsing is therefore performed during the flow of an elution solution throught the porous medium that has been treated, i.e. contacted with the processing material containing the biological product. The skilled person knows based on his or her common general knowledge which elution solution has to be chosen for the applied kind of chromatography separation method and porous medium. The pulsing during the flow of elution solution through the porous medium allows to effectively remove all the mobilized biological product from the porous medium. Without being bound by theory, the yield of biological product is increased by the pulsing because also convetively entrapped biological product can be collected from the porous medium. In one embodiment, the purification method of step b) is a chromatography separation method and the process liquid is a cleaning solution. The skilled person knows based on his or her common general knowledge which cleaning solution has to be chosen for the applied kind of chromatography separation method and porous medium. The pulsing during the flow of cleaning solution through the porous medium after the biological product has been eluted from the porous medium allows to efficiently remove any unwanted material from the porous medium so that the porous medium is optimally regenerated. Without being bound by theory, the pulsing during passing the cleaning solution through the porous medium allows to remove any convectively entrapped unwanted material. In that way, the cleaning of the porous medium is further improved and thus also the lifetime of the porous medium.

In one embodiment, step b.2) is repated at least once with a different process liquid. In that way, the pulsing is used in combination with different process liquids applied during the purification method of step b).

In one embodiment, step b.2) comprises the flow of a washing solution through the treated porous medium of step b.1), wherein pulsing is performed and the flow of an elution solution through the treated porous medium of step b.1), wherein pulsing is performed. In one embodiment, the purification method of step b) is a chromatography separation method and step b.2) comprises the flow of a washing solution through the treated porous medium of step b.1), wherein pulsing is performed and the flow of an elution solution through the treated porous medium of step b.1), wherein pulsing is performed. By applying the pulsing in combination with the use of a washing solution and elution solution, the purity and recovery rate of biological product can be increased.

In one embodiment, step b.2) comprises the flow of an elution solution through the treated porous medium of step b.1), wherein pulsing is performed and the flow of a cleaning solution through the treated porous medium of step b.1), wherein pulsing is performed. In one embodiment, the purification method of step b) is a chromatography separation method and step b.2) comprises the flow of an elution solution through the treated porous medium of step b.1), wherein pulsing is performed and the flow of a cleaning solution through the treated porous medium of step b.1), wherein pulsing is performed. By applying the pulsing in combination with the use of an elution solution and a cleaning solution, the recovery rate of biological product can be increased and at the same time the porous medium can be optimally regenerated.

In one embodiment, step b.2) comprises the flow of a washing solution through the treated porous medium of step b.1), wherein pulsing is performed and the flow of a cleaning solution through the treated porous medium of step b.1), wherein pulsing is performed. In one embodiment, the purification method of step b) is a chromatography separation method and step b.2) comprises the flow of a washing solution through the treated porous medium of step b.1), wherein pulsing is performed and the flow of a cleaning solution through the treated porous medium of step b.1), wherein pulsing is performed. By applying the pulsing in combination with the use of a washing solution and a cleaning solution, the purity of the biological product can be increased and at the same time the porous medium can be optimally regenerated.

In one embodiment, step b.2) comprises the flow of a wahsing solution through the treated porous medium of step b.1), wherein pulsing is performed, the flow of an elution solution through the treated porous medium of step b.1), wherein pulsing is performed and the flow of a cleaning solution through the treated porous medium of step b.1), wherein pulsing is performed. In one embodiment, the purification method of step b) is a chromatography separation method and step b.2) comprises the flow of a wahsing solution through the treated porous medium of step b.1), wherein pulsing is performed, the flow of an elution solution through the treated porous medium of step b.1), wherein pulsing is performed and the flow of a cleaning solution through the treated porous medium of step b.1), wherein pulsing is performed. By applying the pulsing in combination with the use of a washing solution, an elution solution and a cleaning solution, the purity and recovery rate of the biological product can be increased and at the same time the porous medium can be optimally regenerated.

During step b.2), i.e. during the flow of a process liquid through the treated porous medium of step b.1), pulsing is performed. The term "pulse" as used herein refers to a disturbance as defined in options i, ii, iii or iv.

In one embodiment, the pulsing is performed during the entire time the process liquid is passed through the porous medium. In one embodiment, the pulsing is performed during a time interval of the entire time the process liquid is passed through the porous medium.

The pulsing is performed according to one or more of the following options:
i. changing at least two times, preferably periodically, the flow rate of the process liquid through the treated porous medium, wherein the altered flow rate is achieved by allowing the process liquid to flow through a bypass around the treated porous medium;
ii. applying at least two times, preferably periodically, an external force;
iii. switching at least two times, preferably periodically, the process liquid; and
iv. switching at least two times, preferably periodically, two or more parameters of the purification.

In one embodiment, the pulses of option i, ii, iii, or iv have the same length during the pulsing period. The length of the pulses typically depends on the parameter changed during the pulse and its impact on the applied purification method in step b). In one embodiment, a pulse of option i, ii, iii, or iv lasts 1 second, preferably 5 seconds, and more preferably 10 seconds. In one embodiment, the pulses of option i, ii, iii, or iv have different lengths during the pulsing period.

In one embodiment, the pulsing is performed according to option i. Experiments of the inventors have shown that pulsing according to this option is particularly effective in releasing convectively entrapped molecules from a porous medium. In option i, the pulsing is performed by changing at least two times, preferably periodically, the flow rate of the process liquid through the treated porous medium, wherein the altered flow rate is achieved by allowing the process liquid to flow through a bypass around the treated porous medium.

The term "flow rate" as used herein relates to the volumetric flow rate of the process liquid through the treated porous medium. The flow rate thus describes the volume of process liquid that passes per unit time. In a chromatography separation process, the flow rate can, e.g., be indicated in column volumes (CV) per unit of time.

The pulsing based on the flow rate is performed at least two times. This means that the flow rate of the process liquid is changed at least twice during the flow of the process liquid through the treated porous medium. Preferably, the flow rate of the process liquid is changed periordically, i.e. repeatedly over a certain amount in time during the flow of the process liquid through the treated porous medium. The periodic changes can be performed during the entire time the process liquid is passed through the porous medium or only during a certain time period thereof.

In one embodiment, the flow rate is changed between two or more different values. In one embodiment, the flow rate is changed between two different values. In one embodiment, the flow rate is changed between a value of about 0 and a flow rate that is different from a value of about 0. In one embodiment, the method for processing the biological product is a chromatography separation method and the flow rate is changed between a value of about 0 CV/min and a value between 0.1 CV/min to 100 CV/min, preferably a value between 0.5 CV/min to 10 CV/min. In one embodiment, the method for processing the biological product is a chromatography separation method and the flow rate is changed between two different values wherein the two different values are between 0.1 CV/min to 100 CV/min. In one embodiment, the flow rate is changed between a value of about 0 and one or more flow rates that are different from the value of about 0. In one embodiment, the flow rate is changed between a value of about 0 and a flow rate that is different from a value of about 0, wherein the flow rate different from the value of about 0 is gradually increased or decreased during the time the pulsing is performed. In one embodiment, the flow rate is changed between two values different from a value of about 0, wherein the first flow rate is kept constant during the time the pulsing is performed and wherein the second flow rate is gradually increased or decreased during the time the pulsing is performed. Experiments of the inventors have shown that in particular by stopping the flow of process liquid through the porous medium convectively entrapped molecules can be removed efficiently from the porous medium.

In one embodiment, the flow rate is changed between two or more different statuses. In one embodiment, the flow rate is changed between two different statuses. In one embodiment, the flow rate is changed between the status of stopping the flow of process liquid through the porous medium and the status of applying a flow rate of process liquid through the porous medium. In one embodiment, the flow rate is changed between the status of stopping the flow of process liquid through the porous medium and the status of applying one or more flow rates of process liquid through the porous medium. In one embodiment, the flow rate is changed between the status of applying a first flow rate of process liquid through the porous medium and the status of applying a second flow rate of process liquid through the porous medium, wherein the first flow rate and the second flow rate are different from each other. In one embodiment, the flow rate is changed between the status of stopping the flow of process liquid through the porous medium and the status of applying a flow rate of process liquid through the porous medium, wherein the applied flow rate is increased or decreased gradually during the time the pulsing is performed. In one embodiment, the flow rate is changed between the status of applying a first flow rate of process liquid through the porous medium and the status of applying a second flow rate of process liquid through the porous medium, wherein the first flow rate and the second flow rate are different from each other and wherein the second flow rate is increased or decreased gradually during the time the pulsing is performed. Experiments of the inventors have shown that in particular by stopping the flow of process liquid through the porous medium convectively entrapped molecules can be removed efficiently from the porous medium.

As used herein, the term "about" modifying the quantity of a parameter or measurement employed in the method of the invention refers to the variation in the numerical quantity that can occur, for example, through typical measuring procedures in the real world; through inadvertent error in these procedures; through differences in the manufacture, source, or purity of the ingredients employed to carry out the methods; and the like without having a substantial effect on the chemical or physical attributes of the method of the invention. Such variation can be within an order of magnitude, typically within 10%, more typically still within 5%, of a given value or range. Reference to "about" a value or parameter herein includes (and describes) embodiments that are directed to that value or parameter per se. For example, description referring to "about X" includes description of "X".

The alterations of the flow rate during pulsing option i are achieved by allowing the process liquid to flow through a bypass around the treated porous medium. In that way, the flow of the process liquid through the treated porous medium of step b.1) is stopped and resumed at the end of the pulse by directing the flow of process liquid again through the porous medium. The term "bypass" as used herein relates to any means allowing to pass the process liquid around the porous medium to any kind of subsequent processing applied in the purification method of step b). This typcically involves at least a flow through a detector. Using a bypass has several advantages: The bypassing of the process liquid ensures that the flow rate of the process liquid in the subsequent processing steps of the applied purification method is maintained. This is particularly advantegous for subsequent detector measurements. Moreover, a bypass makes it possible that the pump can run constantly without the need of a "ramp-up" phase to establish the desired flow rate. This ensures a constant flow rate in the purification method and thus makes the pulses more precise.

In one embodiment, the pulsing is performed according to option ii. In option ii, the pulsing is performed by applying at least two times, preferably periodically, an external force. The external force during pulsing option ii is applied in such a way that it acts on the flow of the process liquid through the porous medium.

The term "external force" as used herein means any force originating from outside of the purification method applied in step b).

The pulsing based on the external force is performed at least two times. This means that the external force is applied at least twice during the flow of the process liquid through the treated porous medium. Preferably, the external force is applied periordically, i.e. repeatedly over a certain amount in time during the flow of the process liquid through the treated porous medium. The periodic changes can be performed during the entire time the process liquid is passed through the porous medium or only during a certain time period thereof.

In one embodiment, the external force is changed between two or more different values. In one embodiment, the external force is changed between two different values. In one embodiment, the external force is changed between a value of about 0 and a value that is different from the value of about 0. In one embodiment, the external force is changed between a value of about 0 and one or more values that are different from the value of 0. In one embodiment, the external force is changed between a value of about 0 and a value that is different from the value of about 0, wherein the value different from the value of about 0 is gradually increased or decreased during the time the pulsing is performed. In one embodiment, the external force is changed between two values different from the value of about 0, wherein the first value is kept constant during the time the pulsing is performed and wherein the second value is gradually increased or decreased during the time the pulsing is performed.

In one embodiment, the external force is changed between two or more different statuses. In one embodiment, the external force is changed between two different statuses. In one embodiment, the external force is changed between the status of stopping the exposure of the process liquid passing through the porous medium with the external force and the status of performing an exposure of the process liquid passing through the porous medium with the external force. In one embodiment, the external force is changed between the status of applying a first exposure of the process liquid passing through the porous medium with the external force and the status of applying a second exposure of the process liquid passing through the porous medium with the external force, wherein the first exposure and the second exposure are different in strength. In one embodiment, the external force is changed between the status of stopping the exposure of the process liquid passing through the porous medium with the external force and the status of performing one or more different exposures of the process liquid passing through the porous medium with the external force. In one embodiment, the external force is changed between the status of stopping the exposure of the process liquid passing through the porous medium with the external force and the status of performing an exposure of the process liquid passing through the porous medium, wherein the applied external force is increased or decreased gradually during the time the pulsing is performed. In one embodiment, the external force is changed between the status of applying a first exposure of the process liquid passing through the porous medium with the external force and the status of applying a second exposure of the process liquid passing through the porous medium with the external force, wherein the first exposure and the second exposure are different in strength and wherein the second exposure is increased or decreased gradually during the time the pulsing is performed.

In one embodiment, the external force is an electromagnetic field or ultrasound. The skilled person knows based on his or her common general knowledge suitable sources to generate an electromagnetic field or ultrasound.

In one embodiment, the pulsing is performed according to option iii. In option iii, the pulsing is performed by switching at least two times, preferably periodically, the process liquid.

The term "swichting the process liquid" refers to any event in which the previous process liquid is replaced by a different process liquid wherein the difference can be in the type or quantity of the components forming the process liquid.

The pulsing based on the process liquid is performed at least two times. This means that the process liquid is changed at least twice during its flow through the treated porous medium. Preferably, the process liquid is changed periordically, i.e. repeatedly over a certain amount in time during its flow through the treated porous medium. The periodic changes can be performed during the entire time the process liquid is passed through the porous medium or only during a certain time period thereof.

In one embodiment, the process liquid is changed between two different types. In one embodiment, the process liquid is changed between two or more different types.

In one embodiment, the pulsing is performed according to option iv. In option iv, the pulsing is performed by switching at least two times, preferably periodically, two or more parameters of the purification.

The term "parameter" as used herein refers to any parameter relevant for the applied purification method of step b). For example, the parameter can be the flow rate, an external force, the process liquid as used as basis for the pulses of options i, ii, and iii. The parameter changed during the pulse of option iv can, however, also be different from the changed parameters used in the pulses of options i, ii, and iii. In one embodiment, at least one parameter changed in option iv is different from the parameters changed in options i, ii, and iii.

The term "changing two or more parameters" refers to any event in which two or more process parameters are changed quantitatively or qualitatively.

The pulsing based on the change of at least two or more parameters is performed at least two times. This means that two or more parameters are changed at least twice during the flow of the process liquid through the treated porous medium. Preferably, the two or more parameters are changed periordically, i.e. repeatedly over a certain amount in time during the flow of the process liquid through the treated porous medium. The periodic changes can be performed during the entire time the process liquid is passed through the porous medium or only during a certain time period thereof.

In one embodiment, each of the changed two or more parameters is changed between two statuses. In one embodiment, each of the changed two or more parameters is changed between two or more statuses.

In one embodiment, the pulses of options i and ii are applied. In one embodiment, the pulses of options i and iii are applied. In one embodiment, the pulses of options ii and iii are applied.

The method of the invention for processing a biological product comprises at least the following steps:
a) Providing a processing material comprising the biological product;
b) Purifying the processing material of step a) in a chromatography separation method involving
   b.1) a flow of the processing material of step a) through a stationary phase, and
   b.2) a flow of a mobile phase through the treated stationary phase of step b.1), wherein pulsing is performed,
wherein the pulsing of step b.2) is carried out according to one or more of the following options:
i. changing at least two times, preferably periodically, the flow rate of the mobile phase through the treated stationary phase, wherein the altered flow rate is achieved by allowing the mobile phase to flow through a bypass around the treated stationary phase;
ii. applying at least two times, preferably periodically, an external force;
iii. switching at least two times, preferably periodically, the mobile phase; and
iv. switching at least two times, preferably periodically, two or more parameters of the chromatography separation method.

Experiments of the inventors have shown that by using pulses during the flow of the mobile phase through the stationary phase that has been loaded with a desired biological product can be advantageous to increase the overall recovery rate of the biological product, the purity of the biological product and/or the cleaning and thus lifetime of the stationary phase. Pulses according to option i work particularly well in this context.

The method of the invention for processing a biological product comprises at least the following steps:
a) Providing a processing material comprising the biological product;
b) Purifying the processing material of step a) in a chromatography separation method involving
   b.1) a flow of the processing material of step a) through a stationary phase, and
   b.2) a flow of a mobile phase through the treated stationary phase of step b.1), wherein pulsing is performed,
wherein the pulsing of step b.2) is carried out according to one or more of the following options:
i. changing at least two times, preferably periodically, the flow rate of the mobile phase through the treated stationary phase, wherein the altered flow rate is achieved by allowing the mobile phase to flow through a bypass around the treated stationary phase, wherein the flow rate is changed between the status of stopping the flow of process liquid through the porous medium and the status of applying a flow rate of process liquid through the porous medium;
ii. applying at least two times, preferably periodically, an external force;
iii. switching at least two times, preferably periodically, the mobile phase; and
iv. switching at least two times, preferably periodically, two or more parameters of the chromatography separation method.

Experiments of the inventors have shown that by using pulses during the flow of the mobile phase through the stationary phase that has been loaded with a desired biological product can be advantageous to increase the overall recovery rate of the biological product, the purity of the biological product and/or the cleaning and thus lifetime of the stationary phase. Pulses according to option i work particularly well in this context.

The method of the invention for processing a biological product comprises at least the following steps:
a) Providing a processing material comprising the biological product;
b) Purifying the processing material of step a) in a chromatography separation method involving
   b.1) a flow of the processing material of step a) through a stationary phase, and
   b.2) a flow of a mobile phase through the treated stationary phase of step b.1), wherein pulsing is performed,
wherein the pulsing is performed at least partially, preferably completely, during a wash phase, an elution phase, and/or a cleaning phase of the chromatography separation method and wherein the pulsing of step b.2) is carried out according to one or more of the following options:
i. changing at least two times, preferably periodically, the flow rate of the mobile phase through the treated stationary phase, wherein the altered flow rate is achieved by allowing the mobile phase to flow through a bypass around the treated stationary phase;
ii. applying at least two times, preferably periodically, an external force;
iii. switching at least two times, preferably periodically, the mobile phase; and
iv. switching at least two times, preferably periodically, two or more parameters of the chromatography separation method.

Experiments of the inventors have shown that by using pulses during the flow of the mobile phase through the stationary phase that has been loaded with a desired biological product can be advantageous to increase the overall recovery rate of the biological product, the purity of the biological product and/or the cleaning and thus lifetime of the stationary phase. Pulses according to option i work particularly well in this context.

The method of the invention for processing a biological product comprises at least the following steps:
a) Providing a processing material comprising the biological product;
b) Purifying the processing material of step a) in a chromatography separation method involving
   b.1) a flow of the processing material of step a) through a stationary phase, and
   b.2) a flow of a mobile phase through the treated stationary phase of step b.1), wherein pulsing is performed,
wherein the pulsing is performed at least partially, preferably completely, during the elution phase of the chromatography separation method and wherein the pulsing of step b.2) is carried out according to one or more of the following options:
i. changing at least two times, preferably periodically, the flow rate of the mobile phase through the treated stationary phase, wherein the altered flow rate is achieved by allowing the mobile phase to flow through a bypass around the treated stationary phase;
ii. applying at least two times, preferably periodically, an external force;
iii. switching at least two times, preferably periodically, the mobile phase; and
iv. switching at least two times, preferably periodically, two or more parameters of the chromatography separation method.

Experiments of the inventors have shown that by using pulses during elution of the biological product from the stationary phase can be advantageous to increase the overall recovery rate of the biological product from the stationary phase. Pulses according to option i work particularly well in this context.

### The system

The invention also concerns a system configured to perform any embodiment of the method according to the invention.

The advantages described above in connection with the method of the invention apply similarly to the system of the present invention which can be configured to perform any embodiment of the method according to the invention.

### Use

The invention also concerns the use of pulsing in a method for processing a biological product, wherein the method for processing the biological product comprises at least the steps of
a) Providing a processing material comprising the biological product;
b) Purifying the processing material of step a) involving
   b.1) a flow of the processing material of step a) through a porous medium,
      and
   b.2) a flow of a process liquid through the treated porous medium of step b.1), wherein pulsing is performed,
wherein the pulsing of step b.2) is performed according to one or more of the following options
i. changing at least two times, preferably periodically, the flow rate of the process liquid through the treated porous medium, wherein the altered flow rate is achieved by allowing the process liquid to flow through a bypass around the treated porous medium;
ii. changing at least two times, preferably periodically, two or more parameters of the method for processing a biological product;
iii. applying at least two times, preferably periodically, an external force; and
iv. changing at least two times, preferably periodically, the process liquid;
   and wherein the pulsing is used for
   - improving the cleaning of the porous medium;
   - improving the lifetime of the porous medium;
   - improving the purity of the biological product by improving the separation efficiency between the biological product and impurities; and/or
   - improving the recovery of the biological product from the porous medium.

Regarding the method for processing the biological product during which the pulsing is used, the embodiments described above for the method of the invention also apply.

The pulsing in the method for processing the biological product can be used for
- improving the cleaning of the porous medium;
- improving the lifetime of the porous medium;
- improving the purity of the biological product by improving the separation efficiency between the biological product and impurities; and/or
- improving the recovery of the biological product from the porous medium.

The term "improving the cleaning of the porous medium" means that the porous medium is regenerated more effectively and thus contains less unwanted molecules of the previous purification run.

The term "improving the lifetime of the porous medium" means that the porous medium can be used in more purification runs without affecting the quality and quantity of the purified biological product.

The term "improving the purity of the biological product by improving the separation efficiency between the biological product and impurities" means that more pure biological product can be obtained because impurities are separated more efficiently from the biological product.

The term "improving the recovery of the biological product from the porous medium" means that a higher yield of biological product is achieved because more loaded biological product can be extracted from the porous medium.

Without being bound by theory, it is assumed that the pulsing allows the diffusive escape of convetively entrapped molecules. In principle any kind of molecule that has been contacted with the porous medium during the applied purification method in step b) can get convectively entrapped. By applying pulsing durch the flow of process liquid through the porous medium that has been treated with processing material containg a biological product, it is possible to release convetively entrapped biological product and/or unwanted components such as impurities from the porous medium.

The invention also concerns the use of a bypass to enable a pulsing of an applied flow rate during a purification method for purifying a biological product involving a process flow through a porous medium for separation, wherein the bypass allows to guide the process flow around the porous medium.

The term "bypass" as used herein relates to any means allowing to pass the process flow around the porous medium to any kind of subsequent process flow in the purification method. This typcically involves at least a flow through a detector. In one embodiment, the bypass is a multi-port valve.

Using a bypass to enable pulsing in a method for purifying a biological product has several advantages: The bypassing of the process flow around the porous medium ensures that the process flow after the porous medium in the applied purification method can be maintained or kept constant. This is particularly advantegous for subsequent detector measurements. Moreover, a bypass makes it possible that the pump can run constantly without the need of a "ramp-up" phase to establish the desired flow rate. This makes the pulses even more precise so that the pulses can efficiently enable the diffusive escape of convectively entrapped molecules in the porous medium such as the biological product or any kind of other molecule that has been contacted with the porous medium during the purification method.

The pulsing of the flow rate can be performed as described in connection with the method of the invention.

The purification method can be any purification method to purify a biological product which uses a porous medium for separation as described in connection with the method of the invention.

The porous medium can be any porous medium as described in connection with the method of the invention.

### Computer program product

The invention further concerns a computer program product comprising instructions, which, when the program is executed by a computer, cause the computer to carry out at least the step of transmitting a command to a pulsing unit employed in a system configured to perform a method for processing a biological product comprising the steps of
a) Providing a processing material comprising the biological product; and
b) Purifying the processing material of step a) comprising
   b.1) a flow of the processing material of step a) through a porous medium, and
   b.2) a flow of a process liquid through the treated porous medium of step b.1), wherein pulsing is performed,
wherein the command causes the pulsing unit to perform a pulsing according one or more of the following options:
i. switching at least two times, preferably periodically, the flow rate of the process liquid through the treated porous medium, wherein the altered flow rate is achieved by allowing the process liquid to flow through a bypass around the treated porous medium;
ii. switching at least two times, preferably periodically, two or more parameters of the purification;
iii. applying at least two times, preferably periodically, an external force; and
iv. switching at least two times, preferably periodically, the process liquid.

The computer program product according to alternative is suitable for use in the method of the invention.

Additional steps can be included in the computer program product.

The computer program product comprises that a command is transmitted to a pulsing unit employed in a system configured to perform a method for processing a biological product, wherein the command causes the pulsing unit to perform pulsing according to one or more of options i, ii, iii or iv.

The command may be understood as electronic command comprising one or more digital instructions that instruct the pulsing unit to perform the pulsing. For example, the command may comprise a sequence of binary code or machine-level instructions that direct hardware components of the pulisng unit to perform a pulse.

The pulsing unit can be any unit suitable or configured to enable pulsing according to one or more of options i, ii, iii or iv.

In one embodiment, the computer program product comprises program instructions which cause a processor to perform pulsing according to one or more options i, ii, iii or iv when the computer program product runs on the processor. A processor is to be understood, for example, as control unit, microprocessor, microcontrol unit such as a microcontroller, digital signal processor (DSP), application-specific integrated circuit (ASIC) or field programmable gate array (FPGA). In this case, at least the pulsing according to one or more of options i, ii, iii, or iv can be controlled or pulsing according to at least one or more options i, ii, iii, or iv can be performed.

In one embodiment of alternative (i), the computer program is distributable. The distribution of the computer program can take place, for example, via a network such as the Internet, a telephone or mobile radio network and/or a local area network. The computer program can be at least partially software and/or firmware of a processor. The computer program product can be at least partially implemented as hardware.

In one embodiment, the computer program is stored on a storage medium. Such a storage medium can be a computer-readable storage medium such as a magnetic, electrical, optical storage medium and/or any other type of computer-readable storage medium. The storage medium can also be, for example, part of the processor such as a (non-volatile or volatile) program memory of the processor or a part thereof. The storage medium can also be a tangible or physical storage medium.

### EXAMPLES

### Example 1

For separating 15 kbp large plasmid DNA molecules (pFIX15), the following chromatography separation method was used:

| | |
|---|---|
| Column | monolith weak anion exchanger (CIMac pDNA column) with channel size of 1.4 µm in diameter |
| Sample | 15 kbp-large plasmid DNA (pFIX15) with concentration of 30 ug/mL, with 63% supercoiled (SC) and 28% open circular (OC), 5% linear (LIN) pDNA and 4% multimeric (MM) isoforms |
| Loop volume | 200 µL |
| LC system | PATfix^{™} system (Sartorius BIA Separations) with PATfix^{™} 2.1 software |
| Mobile phase A | 100mM TRIS, 0.3M NaCl, pH 8 |
| Mobile phase B | 100mM TRIS, 0.3M guanidinium hydrochloride (GDN) + 0.7M NaCl, pH 8 |
| Gradient | 48-72% Mobile phase B in 12 min |
| Detector | UV at 260 and 360 nm |
| Flow rate | 1 mL/min |
| Pulsating | 10 sec flow rate 1 mL/min, 10 sec flow rate 0; the pulsing was achieved by switching the flow between the column and a bypass |

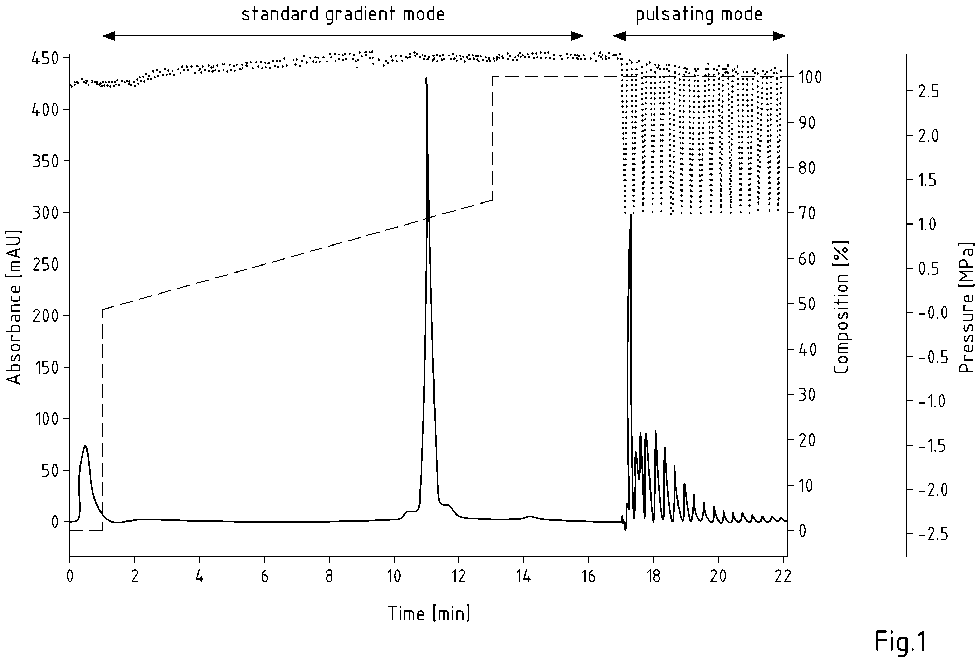

The chromatogram of the chromatographic separation performed according to these parameters is shown in Figure 1.

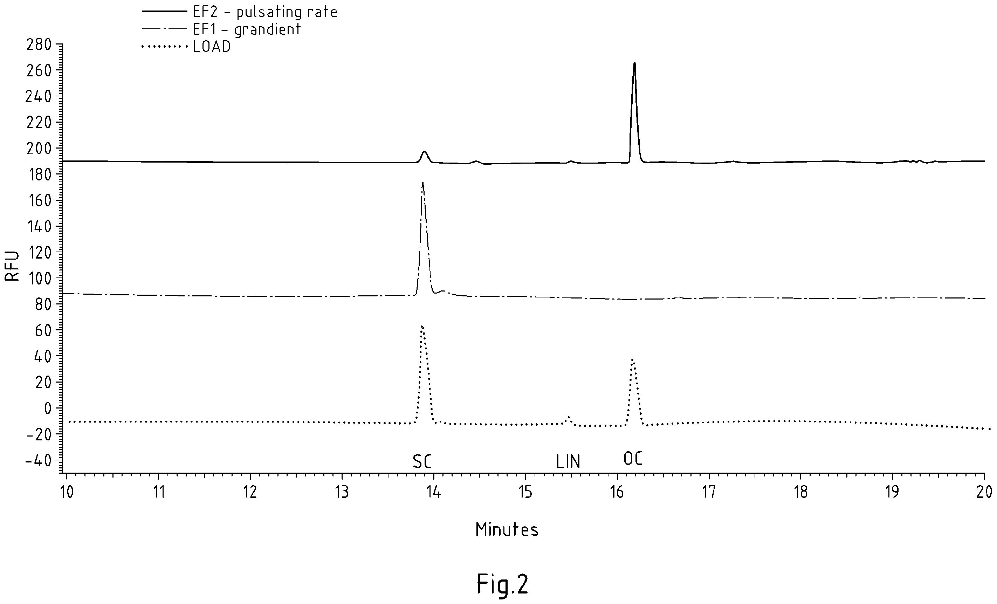

As can be seen from Figure 1, a first pDNA elution was observed during linear gradient with mobile phase B. This fraction was collected ("EF1 - gradient" sample) and analyzed using capillary gel electrophoresis. This analysis demonstrated that the "EF1 - gradient" sample contained 95% supercoiled (SC) pDNA isoform and <1% open coiled (OC) pDNA isoform (see Figure 2). Thus, the "EF1 - gradient" sample lacks OC pDNA and mainly contains SC pDNA.

Once 100% mobile phase B (non-binding conditions) were reached, mobile phase B was applied for 1-2 min. During this time, no additional elution was observed (see Figure 1, time period from 12 min to 16 min). Then, pulsating was turned on. Already after 2 pulses, an increase of the signal was observed (see Figure 1). After 10 pulses, the remaining molecules were completely eluted from the column. This fraction ("EF2 - pulsating flow rate" sample) was collected and also analyzed by capillary gel electrophoresis. This analysis demonstrated that the composition of the "EF2 - pulsating flow rate" sample was 20% SC pDNA isforms and 74% OC pDNA isoforms (besides 2% LIN and 4% MM pDNA). This shows that with the pulsing convectively entrapped OC pDNA isoforms could be eluted.

### Example 2

15 kbp large plasmid DNA molecules (pFIX15) was separated using a CIMac pDNA chromatography column with larger (6 µm) channels as used in Example 1:
The further experimental details of the chromatography separation method were as follows:

| | |
|---|---|
| Column | monolith weak anion exchanger (CIMac pDNA column) with channel size of 6 µm in diameter |
| Sample | 15 kbp-large plasmid DNA (pFIX15) with concentration of 30 ug/mL, containing 63% SC pDNA isoforms, 5% LIN pDNA isoforms, 28% OC pDNA isoforms, and 4 % MM pDNA isoforms. |
| Loop volume | 200 µL |
| LC system | PATfix^{™} system (Sartorius BIA Separations) with PATfix^{™} 2.1 software |
| Mobile phase A | 100mM TRIS, 0.3M GDN, 0.3M NaCl, pH 8 |
| Mobile phase B | 100mM TRIS, 0.3M GDN, 0.7M NaCl, pH 8 |
| Gradient | 7-47% Mobile phase B in 12 min |
| Detector | UV at 260 nm |
| Flow rate | 1 mL/min |
| Pulsating | 12 sec flow rate 1 mL/min, 6 sec flow rate 0 |

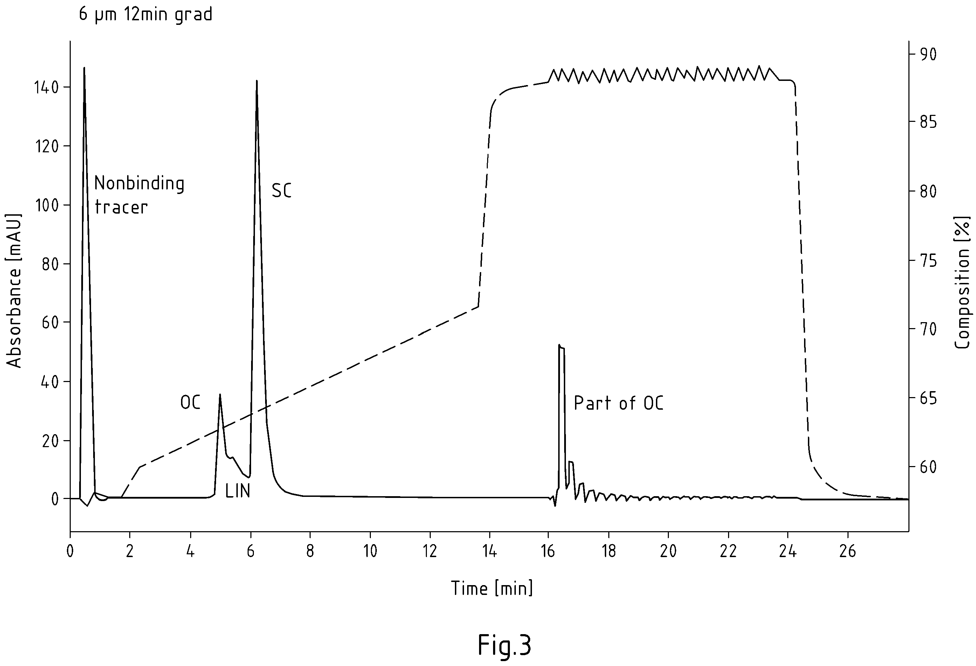

As can be seen from Figure 3 showing the results of the chromatography separation method using a CIMac pDNA with 6 µm channels, a first pDNA elution of LIN pDNA isoforms, OC pDNA isoforms and SC pDNA isoforms was observed during linear gradient with mobile phase B. Once 100% mobile phase B (non-binding conditions) were reached, mobile phase B was applied for 1-2 min. During this time, no additional elution was observed (see Figure 3, time period from 14 min to 16 min). Then, pulsating was turned on. During pulsing, the remaining OC pDNA isforms were completely eluted from the column. Thus, the entrapped OC pDNA isoforms were successfully eluted because of the pulsing.

The relative elution area of OC pDNA between the pulsating and gradient part of the chromatogram is significantly larger for 1.4 µm-channeled column (larger extent of convective entrapment on smaller channels). The comparison of the chromatography separation methods as shown in Figure 1 and 3 further demonstrates that reversible entrapment was higher during chromatography with the CIMac pDNA column having 1.4 µm channels due to narrower constrictions.

### Example 3

15 kbp large plasmid DNA molecules (pFIX15) were separated by applying the following chromatography separation method:

| | |
|---|---|
| Column | CIMac pDNA with 1.4 µm channels |
| Sample | 15 kbp-large plasmid DNA (pFIX15) with a concentration of 52 µg/mL, containing 70% SC pDNA, 25% OC pDNA, and 5% MM pDNA |
| Loop volume | 200 µL |
| LC system | PATfix^{™} system (Sartorius BIA Separations) with PATfix^{™} 2.1 software |
| Mobile phase A | 100mM TRIS, 0.3M GDN, 0.3M NaCl, pH 8 |
| Mobile phase B | 100mM TRIS, 0.3M GDN, 0.7M NaCl, pH 8 |
| Gradient | 7-47% Mobile phase B in 12 min |
| Detector | UV at 260, 280, 320 and 360 nm |
| Flow rate | 1 mL/min |
| Pulsating | 10 sec flow rate 1 mL/min through the column, followed by 10 sec flow bypassing the column. The pulsing was achieved by switching the flow between the column and a bypass - the flow through UV detector was constant |

Pulsation can affect the detector response in a negative way. For example, when the flow rate is stopped during the pulse, the flow stops in the detector cell, consequently increasing the false response. By using a bypass path around the chromatography column which is connected to the main flow path before reaching the detector, the flow rate over the detector is always the same even during the pulses. In preparative runs, a second flow path switch can be introduced after the detector leading the flow directly to the waste container when bypassing the column, to avoid dilution of eluted molecules in the elution fraction. Moreover, a bypass makes it possible that the pump can run constantly without the need of a "ramp-up" phase to establish the desired flow rate. This ensures a constant flow rate and thus makes the pulses more precise.

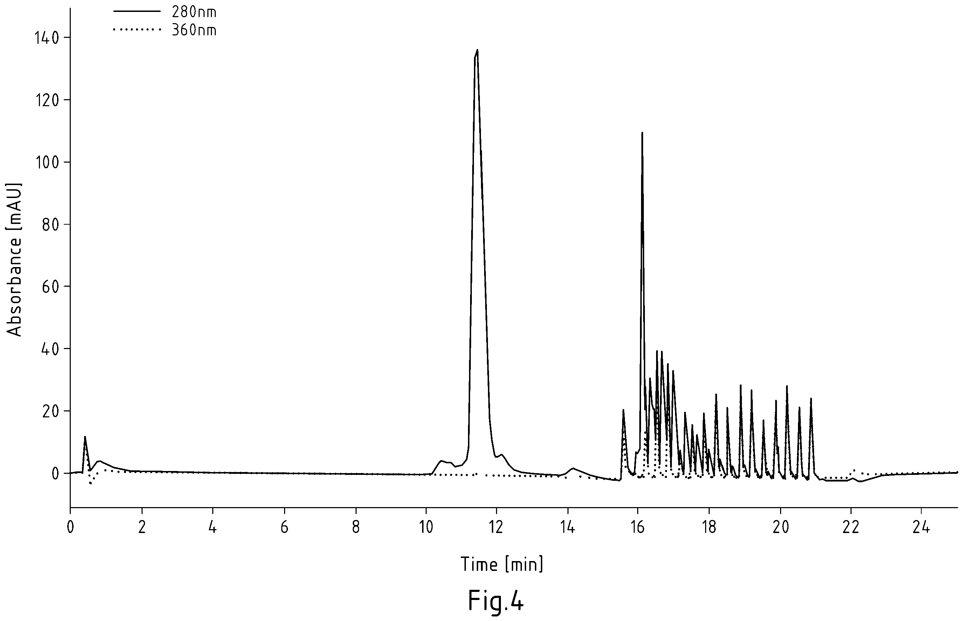

During the chromatography separation method, UV absorption at 280 nm and 360 nm was recorded (see Figure 4). The nucleic acids do not absorb at 360 nm and the signal obtained at this wavelength is just a consequence of physical disturbance, which is identical in size at 280 nm. When subtracting the reference signal at 360 nm from pDNA signal at 280 nm (data not shown), it was possible to extract only the DNA absorbing part of the elution. Peak areas after subtraction showed that 37% of the pDNA was convectively entrapped and could be eluted during the pulsing. With this it was shown that even on-line quantification of biological product during pulsing is possible.

### Example 4

15 kbp large plasmid DNA molecules (pFIX15) were separated by using a CIMac pDNA with 0.5 µm channels. The chromatography separation method was performed with the following settings:

| | |
|---|---|
| Column | monolith weak anion exchanger (CIMac pDNA column) with channel size of 0.5 µm in diameter |
| Sample | 15 kbp-large plasmid DNA (pFIX15) with a concentration of 52 µg/mL, containing 70% SC pDNA, 25% OC pDNA and 5% MM pDNA |
| Loop volume | 200 µL |
| LC system | PATfix^{™} system (Sartorius BIA Separations) with PATfix^{™} 2.1 software |
| Mobile phase A | 100mM TRIS, 0.3M GDN, 0.3M NaCl, pH 8 |
| Mobile phase B | 100mM TRIS, 0.3M GDN, 0.7M NaCl, pH 8 |
| Gradient | 25-48% Mobile phase B in 12 min |
| Detector | UV at 260, 280, 320 and 360 nm |
| Flow rate | 1 mL/min |
| Pulsating | 10 sec flow rate 1 mL/min through the column, 10 sec flow bypassing the column. The pulsing was achieved by switching the flow between the column and a bypass |

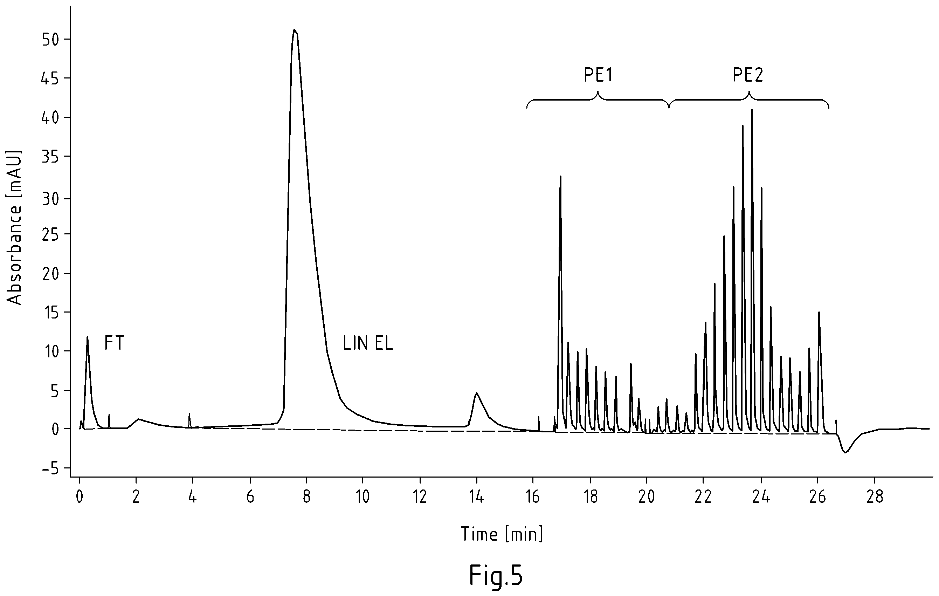

The chromatogram of the chromatographic separation performed according to these parameters is shown in Figure 5.

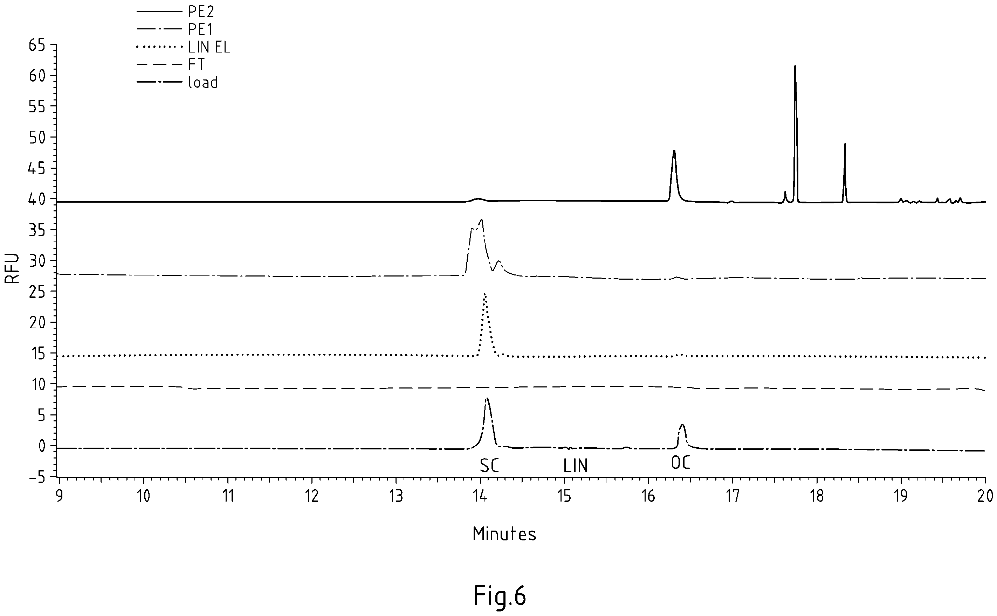

As can be seen from Figure 5, a first pDNA elution was observed during linear gradient with mobile phase B. This fraction was collected ("LIN EL" sample) and analyzed using capillary gel electrophoresis. This analysis demonstrated that the "LIN EL" sample contained highly pure SC pDNA isoforms (see Figure 6).

Once 100% mobile phase B (non-binding conditions) were reached, mobile phase B was applied for 1-2 min. During this time, first a small additional peak appeared (time period from 13.5 to 16 min, which was collected together with LIN EL fraction. Then, pulsating was turned on. During the pulsing fractions, "PE1" (16-20 min) and "PE2" (20-27 min) were collected and analyzed by capillary gel electrophoresis. This analysis demonstrated that the "PE1" sample contained mainly SC pDNA isoforms and that the "PE2" sample contained mainly OC pDNA isoforms. The inventors believe that this is the first time that two pDNA isoforms were separated in isocratic mode on anion exchange column under non-binding conditions.

The amount of eluted pDNA was determined using the chromatographic peak areas (after 360 nm peak subtraction, see the previous Example) in each collected fraction. The results together with the mass balance are summarized in Table 1. 94% of pDNA was successfully recovered from the 0.5 µm-channeled column. This was only possible by performing at least partially pulsing during the chromatographic method.

**Table 1: Mass balance (eluted vs loaded total pDNA) from the chromatogram, shown in Figure 5.**

| Fraction | % of total pDNA in collected fraction |
|---|---|
| Flow through | 2 |
| LIN EL | 64 |
| PE1 | 9 |
| PE2 | 19 |
| Mass balance: | 94% |

### Example 5

15 kbp large plasmid DNA molecules (pFIX15) were loaded onto CIMmic DEAE with 2.0 µm channels (preparative chromatography simulation). The plasmid was then eluted from the column. After elution cleaning-in place (CIP) was performed. The chromatography method was performed with the following settings:

| | |
|---|---|
| Sample | 15 kbp-large plasmid DNA (pFIX15) with a concentration of 18.8 µg/mL pDNA containing 73% SC pDNA, 25% OC pDNA and 2% MM pDNA. |
| pDNA loading amount | Up to full dynamic binding capacity of the column (ca 5 mg pDNA per mL of column) |
| LC system | PATfix^{™} system (Sartorius BIA Separations) with PATfix^{™} 2.1 software |
| Mobile phase A | 50 mM TRIS, 0.2 M NaCl, pH = 7.2 |
| Mobile phase B | 50 mM TRIS, 1 M NaCl, pH = 7.2 |
| Gradient | Step elution with mobile phase B |
| Detector | UV at 260 and 360 nm |
| Flow rate | 1 mL/min during loading, 0.2 mL/min during elution, 0.5mL/min during CIP |
| Pulsating | Experiment A (d05): without pulsing. |
| | Experiment B (d03): pulsing only during the second elution part: 10 sec flow rate 0.2 mL/min through the column, 10 sec flow bypassing the column. The pulsing was achieved by switching the flow between the column and a bypass |
| Cleaning-in-place (CIP) | 0.1 MNaOH, 1 M NaCl |

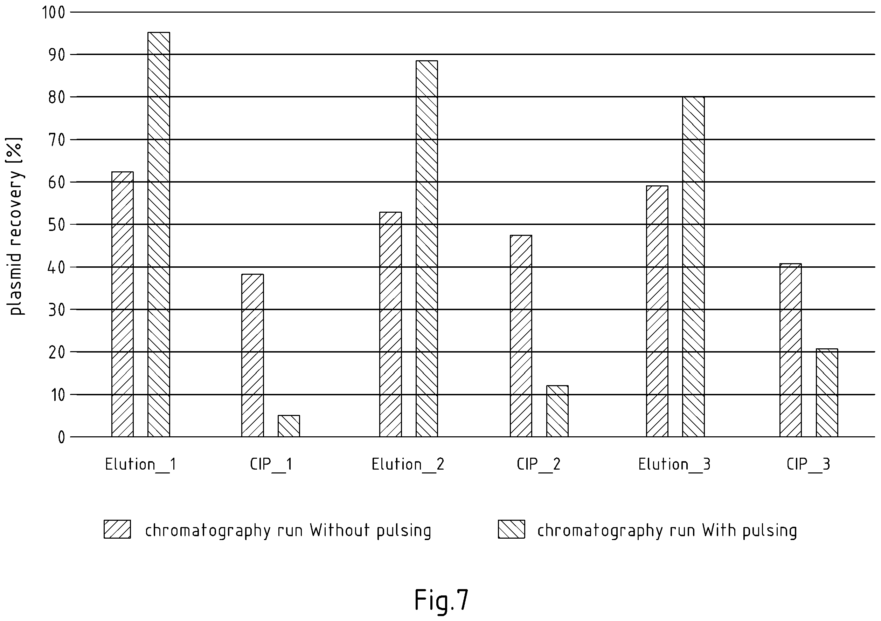

Three consecutive chromatography runs were performed with these settings with or without pulsing. For each chromatography run, the pDNA quantity was determined for the elution fraction and for the CIP fraction. These results showed (see Figure 7) that the total pDNA recovery in the elution fraction was higher during chromatography with pulsing than during chromatography without pulsing. Less total pDNA was therefore detected in the CIP fraction after chromatography with pulsation than after chromatography without pulsing.

### BRIEF DESCRIPTION OF THE DRAWINGS

Figure 1
   Figure 1 shows the results of Example 1. A chromatography separation method was applied to purify 15 kbp large plasmid DNA molecules (channel size of 1.4 µm in diameter). Pulsing was performed at the end of the elution phase. Due to the pulsing, another fraction of pDNA was released from the chromatography column.
Figure 2
   Figure 2 shows the results of the capillary gel electrophoresis that was performed with the fractions of Example 1. This analysis demonstrated that the fraction first eluted with the elution solution contained mainly SC pDNA. In contrast, the fraction eluted with the additional support of the pulsing contained mainly OC pDNA.
Figure 3
   Figure 3 shows the results of Example 2. A chromatography separation method was applied to purify 15 kbp large plasmid DNA molecules (channel size of 6 µm in diameter). During the elution phase, a fraction containing mainly SC pDNA was first eluted. Pulsing was performed at the end of the elution phase. Due to the pulsing, another fraction of pDNA was released from the chromatography column which mainly contained OC pDNA.
Figure 4
   Figure 4 shows the results of Example 3 wherein 15 kbp large plasmid DNA molecules were separated by a chromatography separation method (channel size of 1.4 µm in diameter). Pulsing was performed at the end of the elution phase. By measuring the UV absorption at 280 nm and 360 nm and by subtracting the reference signal at 360 nm from the pDNA signal at 280 nm, it was possible to quantify on line the amount of plasmid DNA released by the pulsing.
Figure 5
   Figure 5 shows the results of Example 4. A chromatography separation method was applied to purify 15 kbp large plasmid DNA molecules (channel size of 0.5 µm in diameter). Pulsing was performed at the end of the elution phase. Due to the pulsing, another fraction of pDNA was released from the chromatography column.
Figure 6
   Figure 6 shows the results of the capillary gel electrophoresis that was performed with the fractions of Example 4. This analysis demonstrated that the fraction first eluted with the elution solution contained mainly SC pDNA. In contrast, the fraction eluted with the additional support of the pulsing contained mainly OC pDNA.
Figure 7
   Figure 7 shows the results of Example 5 (channel size of 2 µm in diameter). Three consecutive chromatography runs were performed with or without pulsing in the elution phase to purify pDNA. The total recovery of pDNA was also way higher during chromatography with pulsing than during chromatography without pulsing. During CIP, less plasmid DNA was therefore detected in the CIP fraction after chromatography with pulsation compared to chromatography without pulsation.
Figure 8

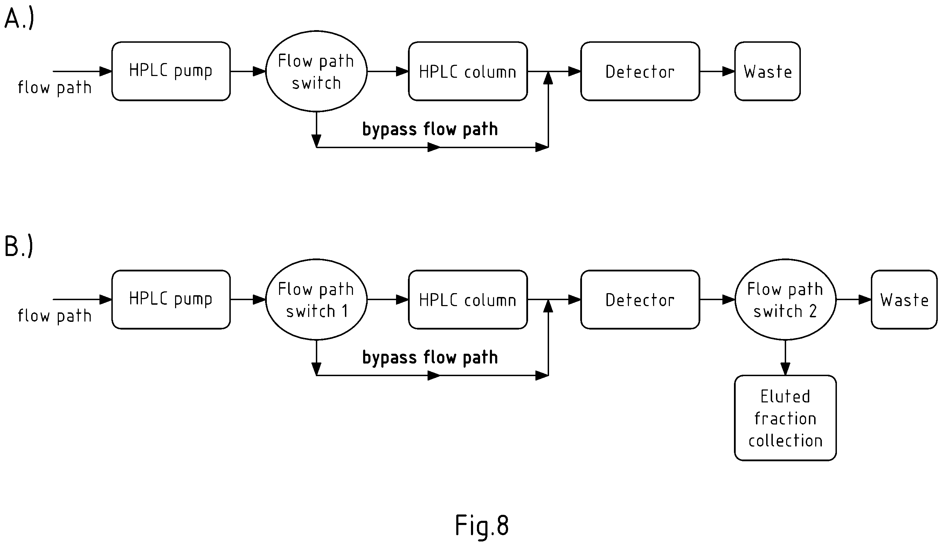

   Figure 8 shows typical set ups of a chromatography separation method to perform the method of the invention. Figure 8A shows a flow path including an HPLC pump, a flow path switch, a HPLC column, a detector and waste container. The flow path switch can allow the flow of the process stream through the bypass around the HPLC column. Figure 8B shows a flow path including an HPLC pump, a flow path switch 1, a HPLC column, a detector, a flow path switch 2, a waste container and a container containing an eluted fraction. The flow path switch 1 can allow the flow of the process stream through the bypass around the HPLC column. The flow path switch 2 can allow the flow of the process stream to the waste container or the container containing the eluted fraction.
Figure 9

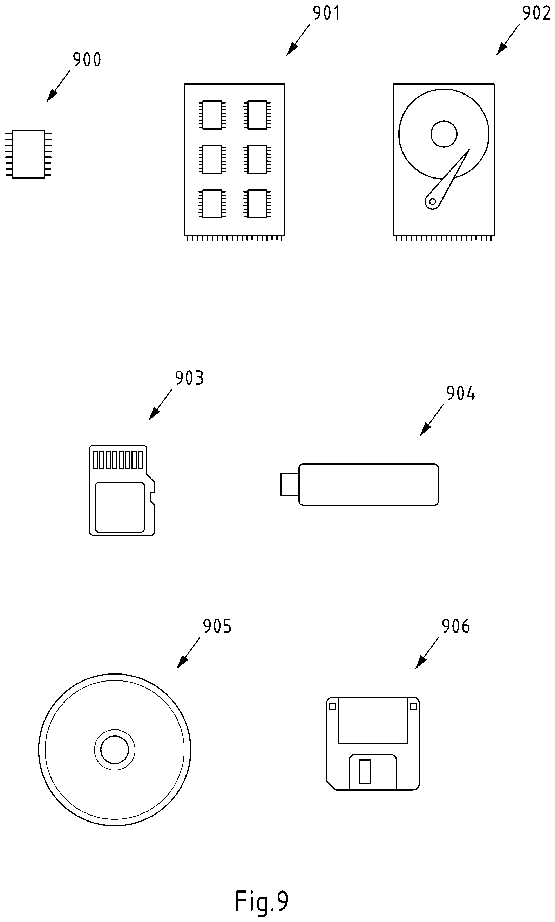

   Figure 9 shows exemplary embodiments of storage media. The storage medium may, by way of example, be a magnetic, electrical, optical and/or other kind of storage medium. The storage medium may, by way of example, be part of a processor, for example a (non-volatile or volatile) program memory of the processor or a part thereof. Exemplary embodiments of a storage medium are a flash memory 900, an SSD hard disc 901, a magnetic hard disc 902, a memory card 903, a memory stick 904 (for example a USB stick), a CD ROM or DVD 905 of a floppy disc 906.
Figure 10

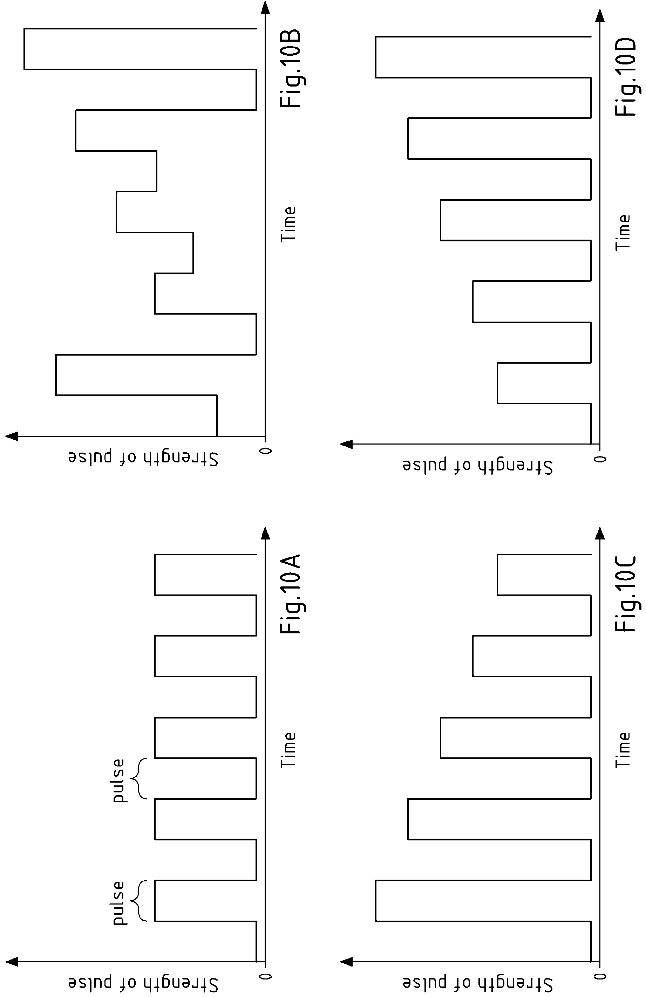

   Figure 10 shows exemplary pulse modi. Figure 10A depicts a pulsing modus wherein two pulse values different from 0 are applied. Figure 10B depicts a pulsing modus wherein 9 different pulse values are applied. Figure 10C depicts a pulsing modus wherein a first pulse value and second pulse value is applied, wherein the first pulse value is different from 0 and is kept constant and wherein the second pulse value is decreased gradually during the time the pulsing is performed. Figure 10D depicts a pulsing modus wherein a first pulse value and second pulse value is applied, wherein the first pulse value is different from 0 and is kept constant and wherein the second pulse value is increased gradually during the time the pulsing is performed.

## Claims

1. Method for processing a biological product comprising at least the following steps:
a) Providing a processing material comprising the biological product;
b) Purifying the processing material of step a) involving
b.1) a flow of the processing material of step a) through a porous medium, and
b.2) a flow of a process liquid through the treated porous medium of step b.1), wherein pulsing is performed,
wherein the pulsing of step b.2) is carried out according to one or more of the following options:
i. changing at least two times, preferably periodically, the flow rate of the process liquid through the treated porous medium, wherein the altered flow rate is achieved by allowing the process liquid to flow through a bypass around the treated porous medium;
ii. applying at least two times, preferably periodically, an external force;
iii. switching at least two times, preferably periodically, the process liquid; and
iv. switching at least two times, preferably periodically, two or more parameters of the purification.

2. Method for processing a biological product according to claim 1, wherein the porous medium is selected from the group consisting of a membrane, a resin, fibers, and a monolithic material.

3. Method for processing a biological product according to claim 1 or 2, wherein the biological product is selected from the group consisting of a nucleic acid, a virus, a bacterium, a eukaryotic cell, a virus-like particle, a cell-derived vesicle, a protein, and mixtures or complexes thereof.

4. Method for processing a biological product according to any one of claims 1 to 3,
wherein the external force according to pulsing option iii is an electromagnetic field or ultrasound.

5. Method for processing a biological product according to any one of claims 1 to 4,
wherein the processing material of step a) is purified in step b) with a chromatography separation method.

6. Method for processing a biological product according to claim 5, wherein the pulsing of step b.2) is performed at least partially during a wash phase, an elution phase, and/or a cleaning phase of the chromatography separation method.

7. Method for processing a biological product according to claim 6, wherein the pulsing is performed during the elution phase.

8. Method for processing a biological product according to claim 5 or 6, wherein the pulsing according to option iv is performed by switching the composition of the mobile phase.

9. System configured to perform the method of claims 1 to 8.

10. Use of pulsing in a method for processing a biological product,
wherein the method for processing the biological product comprises at least the steps of
a) Providing a processing material comprising the biological product;
b) Purifying the processing material of step a) involving
b.1) a flow of the processing material of step a) through a porous medium, and
b.2) a flow of a process liquid through the treated porous medium of step b.1), wherein pulsing is performed,
wherein the pulsing of step b.2) is performed according to one or more of the following options
i. changing at least two times, preferably periodically, the flow rate of the process liquid through the treated porous medium, wherein the altered flow rate is achieved by allowing the process liquid to flow through a bypass around the treated porous medium;
ii. changing at least two times, preferably periodically, two or more parameters of the method for processing a biological product;
iii. applying at least two times, preferably periodically, an external force; and
iv. changing at least two times, preferably periodically, the process liquid;
and wherein the pulsing is used for
- improving the cleaning of the porous medium;
- improving the lifetime of the porous medium;
- improving the purity of the biological product by improving the separation efficiency between the biological product and impurities; and/or
- improving the recovery of the biological product from the porous medium.

11. Use according to claim 10, wherein in the method for processing the biological product the processing material of step a) is purified in step b) by a chromatography separation method, wherein the bypass allows to guide the process flow around the porous medium.

12. Use according to any one of claims 10 or 11, wherein a pulse of option i is used.

13. Use of a bypass to enable a pulsing of an applied flow rate during a purification method for purifying a biological product involving a process flow through a porous medium for separation, wherein the bypass allows to guide the process flow around the porous medium.

14. A computer program product comprising instructions, which, when the program is executed by a computer, cause the computer to carry out at least the step of transmitting a command to a pulsing unit employed in a system configured to perform a method for processing a biological product comprising the steps of
a) Providing a processing material comprising the biological product; and
b) Purifying the processing material of step a) comprising
b.1) a flow of the processing material of step a) through a porous medium, and
b.2) a flow of a process liquid through the treated porous medium of step b.1), wherein pulsing is performed,
wherein the command causes the pulsing unit to perform a pulsing according to one or more of the following options:
i. switching at least two times, preferably periodically, the flow rate of the process liquid through the treated porous medium, wherein the altered flow rate is achieved by allowing the process liquid to flow through a bypass around the treated porous medium;
ii. switching at least two times, preferably periodically, two or more parameters of the purification;
iii. applying at least two times, preferably periodically, an external force; and
iv. switching at least two times, preferably periodically, the process liquid.

15. A computer program product according to claim 14, wherein the system is configured to perform a chromatography separation method for processing the biological product.
